# DEMANDE DE BREVET EUROPEEN

(11) **EP 1 908 494 A1**
(43) Date de publication de la demande: **09.04.2008**
(21) Numéro de dépôt: 07115221.9
(22) Date de dépôt: 29.08.2007
(51) Int. Cl.: A61Q 19/10, A61K 8/04, A61K 8/91, A61K 8/55, A61K 8/60, A61K 8/36, A61K 8/44, A61K 8/46, A61Q 5/02

(54) **Composition de nettoyage contenant des particules hémisphériques creuses**

(30) Priorité: 04.10.2006 FR 0654079
(71) Demandeur: L'Oréal, 75008 Paris (FR)
(72) Inventeur: AUBRUN-SONNEVILLE, Odile, 92160, ANTONY (FR)
(74) Mandataire: Rasson, Catherine

(57) **Abrégé**

La présente invention se rapporte à une composition de nettoyage de la peau ou des cheveux, contenant (1) au moins un tensioactif moussant, (2) des particules concaves ou annulaires de matériau siliconé, notamment sous forme de portions de sphères creuses.

La composition obtenue présente de bonnes propriétés moussantes, et elle donne notamment une mousse fine et douce.

## Description

La présente invention a pour objet une composition pour application topique, contenant des tensioactifs moussants et des particules concaves ou annulaires de matériau siliconé, et l'utilisation de la dire composition dans le domaine cosmétique, en particulier pour le nettoyage des matières kératiniques telles que la peau et les cheveux.

Le nettoyage de la peau est très important pour le soin du visage, et il doit être le plus performant possible car les résidus gras tels que l'excès de sébum, les restes des produits cosmétiques utilisés quotidiennement et les produits de maquillage s'accumulent dans les replis cutanés et peuvent obstruer les pores de la peau et entraîner l'apparition de boutons. Un moyen pour bien nettoyer la peau est d'utiliser des produits de nettoyage moussants. On cherche en général à disposer de produits donnant un volume de mousse important, signe d'une bonne efficacité nettoyante.

Comme produits de nettoyage de la peau, il est connu d'utiliser des gels moussants. Ces produits sont des solutions de tensioactifs moussants utilisés pour leurs propriétés détergentes, ces solutions étant épaissies par des polymères. Ces gels peuvent également contenir des additifs qui permettent d'améliorer la qualité de la mousse, en particulier sa tenue afin d'en faciliter la manipulation, ainsi que sa douceur, synonyme de soin et de respect de la peau pour les utilisateurs qui cherchent à utiliser des produits confortables, qui ne laissent pas de sensation de tiraillements ou de sécheresse. Toutefois, ces additifs sont souvent filmogènes, ce qui peut donner une impression de nettoyage imparfait.

Il subsiste donc le besoin d'une composition de nettoyage, cosmétique et/ou dermatologique, qui soit sous forme de gel et qui à la fois ait de bonnes performances de mousse et soit confortable.

La demanderesse a découvert de façon surprenante que l'utilisation de particules hémisphériques creuses de taille micrométrique dans les gels nettoyants moussants permettait d'améliorer la qualité de la mousse, en particulier sa tenue et sa douceur.

La présente invention a pour objet une composition aqueuse pour application topique contenant (1) au moins un tensioactif moussant, la quantité de tensioactif(s) moussant étant suffisante pour que la composition ait un effet moussant, (2) des particules concaves ou annulaires de matériau siliconé, notamment sous forme de portions de sphères creuses, et (3) au moins 20 % en poids d'eau par rapport au poids total de la composition.

La composition étant destinée à une application topique, elle comprend un milieu physiologiquement acceptable. On entend ici par « milieu physiologiquement acceptable », un milieu compatible avec la peau, les muqueuses, les ongles, le cuir chevelu, les cheveux et/ou les yeux. Il s'agit de préférence d'un milieu cosmétiquement et/ou dermatologiquement acceptable, c'est-à-dire qui, en outre, présente une couleur, une odeur et un toucher agréables et qui ne génère pas d'inconforts inacceptables (picotements, tiraillements, rougeurs), susceptibles de détourner la consommatrice et/ou le consommateur d'utiliser cette composition. La composition de l'invention peut constituer notamment une composition cosmétique ou dermatologique.

Selon un mode préféré de réalisation de l'invention, la composition est exempte d'huile. On entend par « huile » tout composé lipophile liquide à la température ambiante (20-25°C), et on entend par « exempte d'huile » que la composition contient moins de 2 % en poids d'huile et de préférence 0% d'huile.

La composition selon l'invention peut se présenter sous forme de solution, de lait, de crème ou de gel,. La viscosité de la composition, mesurée au moyen d'un appareil Rhéomat 180 à 200 rpm à 25°C, peut aller par exemple de 0.5 à 1000 Pa.s, et de préférence de 0.8 à 500 Pa.s. Le Rheomat 180 est équipé d'un mobile différent selon les viscosités, par exemple d'un mobile 3 pour la gamme des viscosités de 0,2 à 4 Pa.s, et d'un mobile 4 pour la gamme des viscosités supérieures à 2 Pa.s. La viscosité est de préférence mesurée 10 minutes après la mise en rotation du mobile.

Lla composition aqueuse de l'invention comprend un milieu aqueux. Le terme « milieu aqueux » désigne un milieu contenant de l'eau et éventuellement un ou plusieurs solvants organiques hydrosolubles. Dans ce milieu aqueux, la quantité d'eau est d'au moins 20 % en poids par rapport au poids total de la composition. Les compositions selon l'invention peuvent comprendre au moins 30 % en poids, voire au moins 40 % d'eau en poids et de préférence au moins 50 % en poids d'eau par rapport au poids total de la composition. Elles comprennent par exemple de 20 à 90 % en poids, de préférence de 30 à 85 % en poids, mieux de 40 à 80 % en poids par rapport au poids total de la composition.

De façon avantageuse, le pH du milieu aqueux est compatible avec les matières kératiniques et notamment avec la peau. Ce pH va de préférence de 3 à 8,5, mieux de 3,5 à 8, préférentiellement de 4 à 8.

La présence des particules concaves ou annulaires de matériau siliconé permet de conférer à la mousse obtenue par la composition selon l'invention, une tenue et une douceur améliorée, et donc des propriétés de sensation confortable.

L'invention a aussi pour objet un procédé pour améliorer la tenue et la douceur de la mousse obtenue à partir d'une composition pour application topique contenant au moins un tensioactif moussant.

### Particules concaves ou annulaires de matériau siliconé

Les particules concaves ou annulaires présentes dans la composition selon l'invention sont des particules siliconées, en particulier des particules de portions de sphères creuses constituées d'un matériau siliconé.

Lesdites particules ont de préférence un diamètre moyen inférieur ou égal à 10 µm, notamment allant de 0,1 µm à 8 µm, préférentiellement de 0,2 à 7 µm, plus préférentiellement allant de 0,5 à 6 µm, et de préférence encore allant de 0,5 à 4 µm.

Par diamètre moyen, on entend la plus grande dimension de la particule.

De façon avantageuse, ces particules ont une densité supérieure à 1.

Les portions de sphères creuses utilisées dans la composition selon l'invention peuvent avoir la forme de sphères creuses tronquées, présentant un seul orifice communiquant avec leur cavité centrale, et ayant une section transversale en forme de fer à cheval ou d'arceau.

Le matériau siliconé est un polysiloxane réticulé de structure tridimensionnelle ; il comprend de préférence, voire est constitué de, des motifs de formule (1) SiO₂ et de formule (II) R¹SiO_{1,5}
dans lesquels R¹ désigne un groupe organique ayant un atome de carbone directement relié à l'atome de silicium.

Le groupe organique R¹ peut être un groupe organique réactif ; R¹ peut être plus particulièrement un groupe époxy, un groupe (méth)acryloxy, un groupe alkényle, un groupe mercaptoalkyle, aminoalkyle, halogénoalkyle, un groupe glycéroxy, un groupe uréïdo, un groupe cyano, et de préférence, un groupe époxy, un groupe (méth)acryloxy, un groupe alkényle, un groupe mercaptoalkyle, aminoalkyle. Ces groupes comprennent généralement de 2 à 6 atomes de carbone, notamment de 2 à 4 atomes de carbone.

Le groupe organique R¹ peut être aussi un groupe organique non réactif ; R¹ peut être alors plus particulièrement un groupe alkyle en C₁-C₄, notamment un groupe méthyle, éthyle, propyle, butyle, ou un groupe phényle, et de préférence un groupe méthyle.

Comme groupe époxy, on peut citer un groupe 2-glycidoxyéthyle, un groupe 3-glycidoxypropyle, un groupe 2-(3,4-époxycyclohexyl)propyle.

Comme groupe (méth)acryloxy, on peut citer un groupe 3-méthacryloxypropyle, un groupe 3-acryloxypropyle.

Comme groupe alkényle, on peut citer les groupes vinyle, allyle, isopropényle.

Comme groupe mercaptoalkyle, on peut citer les groupes mercaptopropyle, mercaptoéthyle.

Comme groupe aminoalkyle, on peut citer un groupe 3-(2-aminoéthyl)aminopropyle, un groupe 3-aminopropyle, un groupe N,N-diméthylaminopropyle.

Comme groupe halogénoalkyle, on peut citer un groupe 3-chloropropyle, un groupe trifluoropropyle.

Comme groupe glycéroxy, on peut citer un groupe 3-glycéroxypropyle, un groupe 2-glycéroxyéthyle.

Comme groupe uréido, on peut citer un groupe 2-uréidoéthyle.

Comme groupe cyano, on peut citer les groupes cyanopropyle, cyanoéthyle.

De préférence, dans le motif de formule (II), R¹ désigne un groupe méthyle.

Avantageusement, le matériau siliconé comprend les motifs (I) et (II) selon un rapport molaire motif (I) / motif (II) allant de 30/70 à 50/50, de préférence allant de 35/65 à 45/55.

Les particules de matériau siliconé peuvent être notamment susceptibles d'être obtenues selon un procédé comprenant :
(a) l'introduction dans un milieu aqueux, en présence d'au moins un catalyseur d'hydrolyse, et éventuellement d'au moins un tensioactif, d'un composé (III) de formule SiX₄ et d'un composé (IV) de formule RSiY₃, où X et Y désignent indépendamment l'un de l'autre un groupe alcoxy en C₁-C₄, un groupe alcoxyéthoxy renfermant un groupement alcoxy en C₁-C₄, un groupe acyloxy en C₂-C₄, un groupe N,N-dialkylamino renfermant des groupements alkyle en C₁-C₄, un groupe hydroxyle, un atome d'halogène ou un atome d'hydrogène, et R désigne un groupe organique comportant un atome de carbone directement relié à l'atome de silicium ; et
(b) la mise en contact du mélange résultant de l'étape (a) avec une solution aqueuse renfermant au moins un catalyseur de polymérisation et éventuellement au moins un tensioactif, à une température comprise entre 30 et 85°C, pendant au moins deux heures.

L'étape (a) correspond à une réaction d'hydrolyse et l'étape (b) correspond à une réaction de condensation.

Dans l'étape (a), le rapport molaire du composé (III) au composé (IV) va habituellement de 30/70 à 50/50, avantageusement de 35/65 à 45/45, et est préférentiellement de 40/60. Le rapport en poids de l'eau au total des composés (III) et (IV) va de préférence de 10/90 à 70/30. L'ordre d'introduction des composés (III) et (IV) dépend généralement de leur vitesse d'hydrolyse. La température de la réaction d'hydrolyse va généralement de 0 à 40 °C et ne dépasse habituellement pas 30°C pour éviter une condensation prématurée des composés.

Pour les groupements X et Y des composés (III) et (IV) :
Comme groupe alcoxy en C₁-C₄, on peut citer les groupes méthoxy, éthoxy ;
Comme groupe alkoxyéthoxy renfermant un groupe alcoxy en C₁-C₄, on peut citer les groupes méthoxyéthoxy, butoxyéthoxy ;
Comme groupe alcoxy en C₂-C₄, on peut citer les groupes acétoxy, propioxy ;
Comme groupe N,N-dilakylamino renfermant des groupements alkyle en C₁-C₄, on peut citer les groupes diméthylamino, diéthylamino ;
Comme atome d'halogène, on peut citer les atomes de chlore, de brome.

Comme composés de formule (III), on peut citer le tétraméthoxysilane, le tétraéthoxysilane, le tétrabutoxysilane, le triméthoxyéthoxysilane, le tributoxyéthoxysilane, le tétraacétoxysilane, le tétrapropioxysilane, le tétraacétoxysilane, le tétra(diméthylamino)silane, le tétra(diéthylamino)silane, le silane tétraol, le chlorosilane triol, le dichlorodisilanol, le tétrachlorosilane, le chlorotrihydrogénosilane. De préférence, le composé de formule (III) est choisi parmi le tétraméthoxysilane, le tétraéthoxysilane, le tétrabutoxysilane, et leurs mélanges.

Le composé de formule (III) conduit après la réaction de polymérisation à la formation des motifs de formule (I).

Le composé de formule (IV) conduit après la réaction de polymérisation à la formation des motifs de formule (II).

Le groupe R dans le composé de formule (IV) a la signification telle que décrite pour le groupe R¹ pour le composé de formule (II).

Comme exemples de composés de formule (IV) comportant un groupe R organique non réactif, on peut citer le méthyltriméthoxysilane, l'éthyltriéthoxysilane, le propyltributoxysilane, le butyltributoxysilane, le phényltriméthoxyéthoxysilane, le méthyl tributoxyethoxysilane, le méthyltriacétoxysilane, le méthyltripropioxysilane, le méthyltriacétoxysilane, le méthyltri(diméthylamino)silane, le méthyltri(diéthylamino)silane, le méthylsilane triol, le méthylchlorodisilanol, le méthyltrichlorosilane, le méthyltrihydrogénosilane.

Comme exemples de composés de formule (IV) comportant un groupe R organique réactif, on peut citer :
- les silanes ayant un groupe époxy comme le 3-glycidoxypropyl triméthoxysilane, le 3-glycidoxypropyl triéthoxysilane, le 2-(3,4-époxycyclohexyl)éthyl triméthoxysilane, le 3-glycidoxypropylméthyl diméthoxysilane, le 3-glycidoxypropylméthyl diméthoxysilane, le 2-glycidoxyéthylméthyldiméthoxysilane, le 3-glycidoxypropyl diméthylméthoxysilane, le 2-glycidoxyéthyl diméthylméthoxysilane ;
- les silanes ayant un groupe (méth)acryloxy comme le 3-méthacryloxypropyl triméthoxysilane, le 3-acryloxypropyl triméthoxysilane ;
- les silanes ayant un groupe alkényle comme le vinyl triméthoxysilane, l'allyl triméthoxysilane, l'isopropényl triméthoxysilane ;
- les silanes ayant un groupe mercapto comme le mercaptopropyl triméthoxysilane, le mercaptoéthyl triméthoxysilane ;
- les silanes ayant un groupe aminoalkyle comme le 3-aminopropyl triméthoxysilane, le 3-(2-aminoéthyl)aminopropyl triméthoxysilane, le N,N-diméthylaminopropyl triméthoxysilane, le N,N-diméthylaminoéthyl triméthoxy-silane ;
- les silanes ayant un groupe halogénoalkyl comme le 3-chloropropyl triméthoxysilane, le trifluoropropyl triméthoxysilane ;
- les silanes ayant un groupe glycéroxy comme le 3-glycéroxypropyl triméthoxysilane, le di(3-glycéroxypropyl)diméthoxysilane ;
- les silanes ayant un groupe uréido comme le 3-uréïdopropyl triméthoxysilane, le 3-uréïdopropyl méthyldiméthoxysilane, le 3-uréïdopropyl diméthyl-méthoxysilane ;
- les silanes ayant un groupe cyano comme le cyanopropyl triméthoxysilane, le cyanopropyl méthyldiméthoxysilane, le cyanopropyl diméthylméthoxysilane.

De préférence, le composé de formule (IV) comportant un groupe R organique réactif est choisi parmi les silanes ayant un groupe époxy, les silanes ayant un groupe (méth)acryloxy, les silanes ayant un groupe alkényle, les silanes ayant un groupe mercapto, les silanes ayant un groupe aminoalkyle.

Des exemples de composés (III) et (IV) préférés pour la mise en oeuvre de cette invention sont respectivement le tétraéthoxysilane et le méthyltriméthoxysilane.

Comme catalyseurs d'hydrolyse et de polymérisation, on peut utiliser indépendamment des catalyseurs basiques tels que l'hydroxyde de sodium, l'hydroxyde de potassium, le carbonate de sodium, l'hydrogénocarbonate de sodium, l'ammoniaque ou des amines telles que la triméthylamine, la triéthylamine, l'hydroxyde de tétraméthylammonium, ou des catalyseurs acides tels que les acides organiques comme l'acide citrique, l'acide acétique, l'acide méthanesulfonique, l'acide p-toluène sulfonique, l'acide dodécylbenzène-sulfonique, l'acide dodécylsulfonique, ou les acides minéraux tels que l'acide chlorhydrique, l'acide sulfurique, l'acide phosphorique.

Lorsqu'il est présent, le tensioactif utilisé est de préférence un tensioactif non ionique ou anionique ou un mélange des deux. Le dodécylbenzènesulfonate de sodium peut être utilisé comme tensioactif anionique. La fin de l'hydrolyse est marquée par la disparition des produits (III) et (IV), insolubles dans l'eau, et l'obtention d'une couche liquide homogène.

L'étape (b) de condensation peut utiliser le même catalyseur que l'étape d'hydrolyse ou un autre catalyseur choisi parmi ceux mentionnés précédemment.

A l'issue de ce procédé, on obtient une suspension dans l'eau de fines particules organosiliconées qui peuvent éventuellement être ensuite séparées de leur milieu. Le procédé décrit ci-dessus peut donc comprendre une étape supplémentaire de filtration, par exemple sur filtre à membrane, du produit résultant de l'étape (b), suivie éventuellement d'une étape de centrifugation du filtrat destinée à séparer les particules du milieu liquide, puis d'une étape de séchage des particules. D'autres méthodes de séparation peuvent bien entendu être employées.

La forme des portions de sphères creuses obtenues selon le procédé ci-dessus, ainsi que leurs dimensions, dépendront notamment du mode de mise en contact des produits à l'étape (b).

Un pH plutôt basique et une introduction à froid du catalyseur de polymérisation dans le mélange issu de l'étape (a) conduira à des portions de sphères creuses en forme de "bols" à fond arrondi, tandis qu'un pH plutôt acide, et une introduction goutte-à-goutte du mélange issu de l'étape (a) dans le catalyseur de polymérisation chaud, conduira à des portions de sphères creuses ayant une section transversale en forme de "fer à cheval".

Selon un mode de réalisation préféré de l'invention, on utilise des portions de sphères creuses en forme de "bols". Celles-ci peuvent être obtenues comme décrit dans la demande JP-A-2003-128788.

Des portions de sphères creuses en forme de fer à cheval sont décrites dans la demande JP-A-2000-191789.

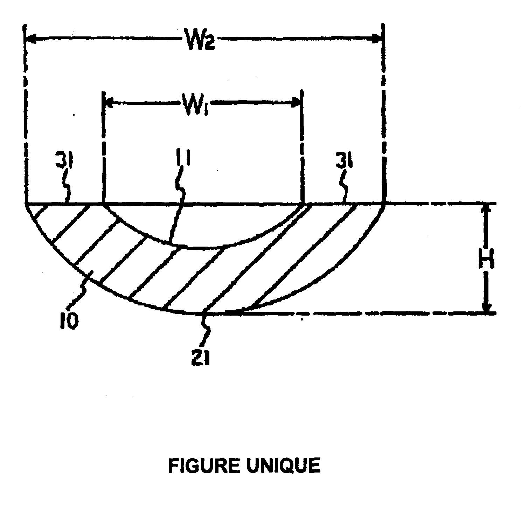

La figure 1 annexée illustre une particule concave sous forme de portions de sphères en forme de bol en coupe transversale. La largeur W2 correspond au diamètre des particules.

Comme il ressort de cette figure, ces portions concaves sont formées (en coupe perpendiculaire à un plan de l'ouverture délimitée par la portion de sphère creuse) d'un petit arc interne (11), d'un grand arc externe (21) et de segments (31) qui relient les extrémités des arcs respectifs, la largeur (W1) entre les deux extrémités du petit arc interne (11) allant de 0,01 à 8 µm, de préférence de 0,02 à 6 µm en moyenne, la largeur (W2) entre les eux extrémités du grand arc externe (21) allant de 0,05 à 10 µm, de préférence de 0,06 à 8 µm en moyenne et la hauteur (H) du grand arc externe (21) allant de 0,015 à 8 µm, de préférence de 0,03 à 6 µm en moyenne.

Les dimensions mentionnées ci-dessus sont obtenues en calculant la moyenne des dimensions de cent particules choisies sur une image obtenue au microscope électronique à balayage.

Comme particules concaves sous forme de portions de sphères utilisables selon l'invention, on peut citer par exemple :
- les particules constituées de l'organosilicone réticulé TAK-110 (polymère réticulé méthylsilanol/silicate) de la société TAKEMOTO OIL & FAT, en forme de bol, de largeur 2,5 µm, de hauteur 1,2 µm et d'épaisseur 150 nm (particules vendues sous la dénomination NLK-506 par la société Takemoto Oil & Fat) ;
- les particules constituées de l'organosilicone réticulé TAK-110 (polymère réticulé méthylsilanol/silicate) de la société TAKEMOTO OIL & FAT, en forme de bol, de largeur 0,8 µm, de hauteur 0,4 µm et d'épaisseur 130 nm (particules vendues sous la dénomination NLK-515 par la société Takemoto Oil & Fat) ;
- les particules constituées de l'organosilicone réticulé TAK-110 (polymère réticulé méthylsilanol/silicate) de la société TAKEMOTO OIL & FAT, en forme de bol, de largeur 7 µm, de hauteur 3,5 µm et d'épaisseur 200 nm (particules vendues sous la dénomination NLK-51 0 par la société Takemoto Oil & Fat).

Ces particules ont pour nom INCl: Methylesilanol/silicate crosspolymer.

Avantageusement, les particules concaves siliconées ont un diamètre moyen inférieur ou égal à 5 µm, notamment allant de 0,1 µm à 5 µm, préférentiellement allant de 0,2 à 5 µm, plus préférentiellement allant de 0,5 à 4 µm, et de préférence encore allant de 0,5 à 3 µm.

Ces particules permettent, outre la réduction voire l'élimination du toucher collant, l'optimisation des propriétés de glissant, d'étalement, et de confort de la composition selon l'invention.

Les particules siliconées de forme annulaire sont de préférence choisies parmi celles décrites et synthétisées dans la demande US-A-2006/0089478. Elles présentent un diamètre moyen extérieur de 0,05 à 15 µm et un diamètre moyen intérieur de 001 à 10 µm; la différence entre le diamètre moyen extérieur et le diamètre moyen intérieur étant de 0,04 à 5 µm.

Elles présentent un réseau polysiloxane comprenant des unités siloxane de formule (1), (2) (3) (4) (5) et (6)

SiO_{4/2} (1)

Si(OH)_{3/2} (2)

R'SiO_{3/2} (3)

R²SiO_{3/2} (4)

R³(SiOH)_{2/2} (5)

R⁴(SiOH)_{2/2} (6)

dans lesquelles
- R¹ et R³ désignent des groupes hydrocarbonés non réactifs, notamment des groupes alkyle, cycloalkyle, aryle, alkylaryle ou aralkyle, de préférence les groupes alkyles en C₁-C₃, notamment méthyle, éthyle, propyle, et préférentiellement un groupe méthyle,
- R² et R⁴ désignent chacun un groupe hydrocarboné choisi parmi les groupes acryloxy, methacryloxy, vinyle ou mercapto ;
le rapport molaire unités siloxane de formule (1) / unités siloxane de formule (2), (3), (4), (5) et (6) étant de 20/80 à 50/50 ;
le rapport molaire unités siloxane de formule (2), (3) et (4) / unités siloxane de formule (5) et (6) étant de 50/50 à 75/25;
le rapport molaire unités siloxane de formule (3) et (5) / unités siloxane de formule (4) et (6) étant de 20/80 à 60/40.

Comme groupe acryloxy, on peut citer un groupe 2-méthacryloxyéthyle, un groupe 3-acryloxypropyl.

Comme groupe (méth)acryloxy, on peut citer un groupe 3-méthacryloxypropyl, un groupe 3-acryloxypropyle.

Comme groupe mercaptoalkyle, on peut citer un groupe mercaptopropyl, mercaptoéthyl.

Comme groupe vinyle, on peut citer les groupes allyle, isopropenyle, 2-methylallyle.

Les particules concaves ou annulaires siliconées peuvent être présentes dans la composition selon l'invention en une teneur allant de 0,1 à 20 % en poids et de préférence allant de 0,5 à 10 % en poids par rapport au poids total de la composition.

### Tensioactifs moussants

La composition de nettoyage selon l'invention comprend un ou plusieurs tensioactifs moussants, qui vont apporter le caractère nettoyant à la composition. Ce ou ces tensioactifs moussants peuvent être choisis parmi les tensioactifs non ioniques, les tensioactifs anioniques, les tensioactifs cationiques, les tensioactifs amphotères et zwitterioniques et les mélanges de ceux-ci.

Les tensioactifs moussants sont des détergents et se différencient des tensioactifs émulsionnants par la valeur de leur HLB (Hydrophilic Lipophilic balance), le HLB étant le rapport entre la partie hydrophile et la partie lipophile dans la molécule. Le terme HLB est bien connu de l'homme du métier et est décrit par exemple dans "The HLB system. A time-saving guide to Emulsifier Selection" (publié par ICI Americas Inc ; 1984).

Pour les tensioactifs émulsionnants, le HLB va généralement de 3 à 8 pour la préparation des émulsions eau-dans-huile (E/H) et de 8 à 18 pour la préparation des émulsions huile-dans-eau (H/E), alors que les tensioactifs moussants ont généralement un HLB supérieur à 18 et mieux supérieur à 20.

Le ou les tensioactifs moussants doivent être en quantité suffisante pour que la composition ait un effet moussant. La quantité (en matière active) de tensioactif(s) moussant(s) dans la composition selon l'invention peut aller par exemple de 1 à 50 % en poids, de préférence de 2 à 40 % en poids, en particulier de 2 à 35 % en poids, plus spécialement de 5 à 30 % en poids, voire de 5 à 25 % en poids par rapport au poids total de la composition.

a) Les tensioactifs non ioniques peuvent être choisis par exemple parmi les alkyl polyglucosides (APG), les esters de maltose, les alcools gras polyglycérolés, les dérivés de glucamine comme l'éthyl-2 hexyl oxy-carbonyl n-méthyl glucamine, et leurs mélanges.

Comme alkylpolyglucosides, on utilise de préférence ceux contenant un groupe alkyle comportant de 6 à 30 atomes de carbone et de préférence de 8 à 16 atomes de carbone, et contenant un groupe hydrophile (glucoside) comprenant de préférence 1, 2 à 3 unités de glucoside. Comme alkylpolyglucosides, on peut citer par exemple le décylglucoside (Alkyl-C9/C11-polyglucoside (1.4)) comme le produit commercialisé sous la dénomination MYDOL 10^{®} par la société Kao Chemicals, le produit commercialisé sous la dénomination PLANTAREN 2000 UP^{®} par la société Cognis, et le produit commercialisé sous la dénomination ORAMIX NS 10^{®} par la société Seppic ; le caprylyl/capryl glucoside comme le produit commercialisé sous la dénomination ORAMIX CG 110^{®} par la Société Seppic ; le laurylglucoside comme les produits commercialisés sous les dénominations PLANTAREN 1200 N^{®} et PLANTACARE 1200^{®} par la société Cognis ; le coco-glucoside comme le produit commercialisé sous la dénomination PLANTACARE 818/UP^{®} par la société Cognis ; et leurs mélanges.

Les dérivés de maltose sont par exemple ceux décrits dans le document EP-A-566438, tels que l'O-octanoyl-6'-D-maltose, ou encore le O-dodecanoyl-6'-D-maltose décrit dans le document FR-2,739,556.

Parmi les alcools gras polyglycérolés on peut citer le dodecanediol polyglycérolé (3,5 moles de glycérol), produit commercialisé sous la dénomination CHIMEXANE NF^{®} par la société Chimex.

b) Les tensioactifs anioniques peuvent être choisis par exemple parmi les savons (sels d'acides gras), les carboxylates, les acylaminoacides, les amidoéther-carboxylates, les alkyl polyaminocarboxylates, les alkyl éthers sulfates tels que les Sodium Laureth sulfates, les alkyl sulfonates, les iséthionates, les alkyl méthyltaurates, les alkyl sulfosuccinates, les alkylsulfoacétates, les alkylphosphates (mono ou dialkylphosphates), leurs sels, et leurs mélanges.

Les savons sont obtenus à partir d'un acide gras qui est partiellement ou totalement saponifié (neutralisé) par un agent basique. Ce sont des savons de métal alcalin ou alcalino-terreux ou de bases organiques. Comme acides gras, on peut utiliser les acides gras saturés, linéaires ou ramifiés, comportant de 8 à 30 atomes de carbone, et de préférence comportant de 8 à 22 atomes de carbone. Cet acide gras peut être en particulier choisi parmi l'acide palmitique, l'acide stéarique, l'acide myristique, l'acide laurique et leurs mélanges.

Comme agents basiques, on peut utiliser par exemple les hydroxydes de métaux alcalins (hydroxyde de sodium et hydroxyde de potassium ou potasse), les hydroxydes de métaux alcalino-terreux (par exemple de magnésium), l'hydroxyde d'ammonium, ou encore les bases organiques comme la triéthanolamine, la N-méthylglucamine, la lysine et l'arginine.

Les savons peuvent être notamment des sels alcalins d'acide gras, l'agent basique étant un hydroxyde de métal alcalin, et de préférence l'hydroxyde de potassium ou potasse (KOH).

La quantité d'agent basique doit être suffisante pour que l'acide gras soit au moins partiellement neutralisé.

Comme carboxylates, on peut citer notamment les acides alkyl glycol carboxyliques (ou acides 2-(2-Hydroxyalkyloxy acétique), et leurs sels tels que par exemple le lauryl glycol carboxylate de sodium, commercialisé sous les dénominations BEAULIGHT SHAA^{®} ou BEAULIGHT LCA-25N^{®} par la société SANYO (nom CTFA : Sodium Lauryl Glycol Carboxylate), ou sa forme acide correspondante commercialisée sous la dénomination BEAULIGHT SHAA (Acid Form)^{®} par la société SANYO.

Comme acylaminoacides, on peut citer par exemple le cocoylglycinate de sodium commercialisé par la société Ajinomoto sous la dénomination Amilite GCS 12, le lauroyl glutamate de sodium commercialisé par la société Ajinomoto sous la dénomination Amisoft LS11 et le lauroyl sarcosinate de sodium commercialisé par la société Seppic sous la dénomination ORAMIX L 30.

Comme alkylphosphates, on peut citer par exemple le laurylphosphate commercialisé par la société Kao sous la dénomination MAP 20.

c) Les tensioactifs moussants amphotères et zwitterioniques peuvent être choisis par exemple parmi les dérivés de bétaïnes dont les amidopropylbétaïnes, les amphoacétates et les amphodiacétates, les hydroxylsultaines et leurs mélanges.

Comme dérivés de bétaïnes, on peut citer par exemple la cocobétaïne comme le produit commercialisé sous la dénomination DEHYTON AB-30^{®} par la société Cognis ; la laurylbétaïne comme le produit commercialisé sous la dénomination GENAGEN KB^{®} par la société Clariant ; la laurylbétaïne oxyethylénée (10 OE), comme le produit commercialisé sous la dénomination LAURYLETHER(10 OE)BETAINE^{®} par la société Shin Nihon Rica; la stéarylbétaïne oxyéthylénée (10 OE) comme le produit commercialisé sous la dénomination STEARYLETHER(10 OE)BETAINE^{®} par la société Shin Nihon Rica ; la cocamidopropyl betaïne commercialisé par exemple sous la dénomination VELVETEX BK 35^{®} par la société Cognis ; le undecylenamidopropyl betaïne commercialisé par exemple sous la dénomination AMPHORAM U^{®} par la société Ceca ; et leurs mélanges.

Comme amphoacétates et amphodiacétates, on peut citer par exemple le N-cocoyl-N-carboxyméthoxyéthyl-N-carboxyméthyl-éthylènediamine N-di-sodique (nom CTFA: disodium cocamphodiacetate) comme le produit commercialisé sous la dénomination MIRANOL C2M CONCENTRE NP^{®} par la société Rhodia Chimie ; le N-cocoyl-N-hydroxyéthyl-N-carboxyméthyl-éthylènediamine N-sodique (nom CTFA : sodium cocamphoacetate) et leurs mélanges.

Selon un mode de réalisation de l'invention, un tensioactif convenant à l'invention peut être choisi parmi les alkylpolyglucosides, les dérivés de bétaïne, les acides alkyl glycol carboxyliques et leurs sels, les savons, les alkyl éthers sulfates, les alkylphosphates, les amphodiacétates, les amphoacétates, les alkylglycinates, les acylglutamates, les acylsarcosinates et leurs mélanges.

Selon un mode particulier de réalisation de l'invention, le tensioactif moussant peut être choisi notamment parmi le décyl glucoside, le cocoyl glucoside, le lauryl éthersulfate de sodium, le cocoyl bétaïne, la lauroyl bétaïne, la cocamidopropyl bétaïne, la lauramido propylbétaïne, le lauryl glycol carboxylate, le cocoampho(di)acétate, le lauroampho(di)acétate, le lauryl phosphate de potassium, les sels d'acide laurique, les sels d'acide myristique, les sels d'acide stéarique, les sels d'acide palmitique, et leurs mélanges.

### Adjuvants

De façon connue, la composition de l'invention peut contenir également des adjuvants et actifs classiquement utilisés dans le domaine cosmétique ou dermatologique, On peut citer par exemple les adjuvants hydrosolubles ou liposolubles tels que les polymères hydrophiles ou lipophiles, anioniques, non ioniques, cationiques ou amphotères, épaississants, dispersants ou conditionneurs ; les conservateurs ; les séquestrants (EDTA) ; les antioxydants, les parfums ; les matières colorantes telles que les colorants solubles ou les pigments encapsulés ou non ; les nacres ; les charges à effets matifiant, tenseur, blanchissant ou exfoliant ; les filtres solaires ; les actifs cosmétiques ou dermatologiques hydrophiles ou lipophiles tels que les vitamines hydrosolubles ou liposolubles, les antiseptiques, les antiséborrhéïques, les antimicrobiens tels que le peroxyde de benzoyle, l'acide salicylique, le triclosan, l'acide azélaïque, la niacine (vit. PP) ; les amincissants tels que la caféine et aussi les azurants optiques ; les électrolytes ; les agents ayant pour effet d'améliorer les propriétés cosmétiques de la peau ou encore des particules solides sphériques ou non, poreuses ou non de toute taille.

Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse ou dans la phase aqueuse.

Bien entendu, l'homme de l'art veillera à choisir le ou les éventuels additifs à ajouter à la composition selon l'invention et leurs quantités, de manière telle que les propriétés avantageuses attachées intrinsèquement à la composition conforme à l'invention ne soient pas, ou substantiellement pas, altérées par l'addition envisagée.

Comme charges exfoliantes, la composition peut contenir des particules exfoliantes ou gommantes d'origine minérale, végétale ou organique. Ainsi, on peut utiliser par exemple des billes ou de la poudre de polyéthylène, de la poudre de nylon, de la poudre de polychlorure de vinyle, de la pierre ponce, des broyats de noyaux d'abricots ou de coques de noix, de la sciure de bois, des billes de verre, l'alumine, et leurs mélanges. Ces particules peuvent être présentes en une quantité allant par exemple de 0,5 à 30 % en poids, de préférence de 1 à 20 % en poids et mieux de 1 à 10 % en poids par rapport au poids total de la composition. Quand la composition contient des particules exfoliantes, elle peut constituer notamment une composition de gommage de la peau du visage ou du corps.

La composition selon l'invention peut constituer en particulier une composition cosmétique de nettoyage des matières kératiniques, notamment de la peau et des cheveux, et notamment une composition cosmétique moussante qui peut notamment être rincée après application sur la peau ou les cheveux.

L'invention a aussi pour objet, l'utilisation cosmétique de la composition telle que définie ci-dessus, pour le nettoyage des matières kératiniques, notamment de la peau et des cheveux, et tout particulièrement de la peau.

L'invention a encore pour objet, un procédé cosmétique de nettoyage de la peau, consistant à appliquer sur la peau, une composition telle que définie ci-dessus, et à rincer la peau.

Les exemples qui suivent servent à illustrer l'invention sans toutefois présenter un caractère limitatif. Les quantités sont exprimées en pourcentage en poids de matière première. Les composés des compositions selon l'invention sont, selon le cas, cités en noms chimiques ou en noms INCl (International Cosmetic Ingredient).

### Exemple 1 selon l'invention et exemple comparatif 1

| | Exemple 1 selon l'invention | Exemple comparatif 1 |
|---|---|---|
| Lauryl phosphate (1) | 6.5 | 6.5 |
| Décyl glucoside (2) | 6.5 | 6.5 |
| Polyquaternium-7 (3) | 0.5 | 0.5 |
| PEG-60 hydrogenated castor oil (4) | 0.2 | 0.2 |
| PEG-200 glyceryl stearate (5) | 0.5 | 0.5 |
| Potasse (KOH) | 1.7 | 1.7 |
| Chlorure de sodium | 0.1 | 0.1 |
| EDTA | 0.05 | 0.05 |
| Sorbitol | 3.5 | 3.5 |
| Imidazolidinyl urée | 0.2 | 0.2 |
| Méthyl paraben | 0.2 | 0.2 |
| Glycérine | 3.5 | 3.5 |
| Methylsilanol/silicate crosspolymer (6) | 2.5 | - |
| Eau | Qsp 100 % | Qsp 100 % |

| | | |
|---|---|---|
| (1) MAP 20® de la société Kao Chemicals (2) MYDOL 10® de la société Kao Chemicals (3) MERQUAT S® de la société Calgon (4) CREMOPHOR RH 60 de la société BASF (5) SIMULSOL 220 TM de la société Seppic (6) NLK 506 de la société Takemoto Oil&fat | | |

### Performances sensorielles

La qualité de la mousse développée a été évaluée selon le protocole décrit ci-dessous.

Avant toute utilisation du produit, les mains sont lavées au savon de Marseille puis convenablement rincées et séchées. Puis, le protocole appliqué est le suivant :
1- mouiller les mains en les passant sous l'eau courante, les secouer trois fois pour les essorer
2- placer 1 g de produit dans le creux de l'une des mains
3- travailler le produit entre les deux paumes pendant 10 secondes,
4- ajouter 2 ml d'eau et travailler le produit à nouveau pendant 10 secondes,
5- ajouter 2 ml d'eau et travailler le produit à nouveau pendant 10 secondes
6- évaluer la qualité de la mousse selon les critères définis
7- rincer les mains sous l'eau
8- les essuyer.

Les critères de qualité de mousse (étape 6) sont notés sur une échelle de 0 à 10 :
- *Le volume de mousse :* la note attribuée est d'autant plus élevée que le volume est grand.
- *La taille des bulles composant la mousse* : la note attribuée est d'autant plus élevée que les bulles sont grosses.
- *La tenue de la mousse :* la note attribuée est d'autant plus élevée que la mousse est plus élastique
- *La douceur de la mousse* : la note attribuée est d'autant plus élevée que la mousse est glissante et douce

Le panel d'évaluation était constitué de 3 experts entraînés, et on a fait la moyenne des trois notes, ce qui a permis de comparer les compositions selon chacun des critères.

| | Exemple selon l'invention | Exemple comparatif |
|---|---|---|
| Qualité de mousse | | |
| Volume | 7 | 6.3 |
| Taille des bulles | 3.7 | 4.2 |
| Tenue | 7.5 | 6.5 |
| Douceur de la mousse | 9.3 | 8.7 |

Le tableau ci-dessous montre que l'exemple selon l'invention qui contient des sphères creuses organosiliconées, présente une qualité de mousse supérieure, en particulier une mousse plus dense, plus douce et légèrement plus fine (taille des bulles plus petite) et plus volumineuse.

### Exemple 2 selon l'invention : crème savon

| | Exemple 2 |
|---|---|
| Acide laurique | 3 |
| Acide myristique | 20 |
| Acide stéarique | 6 |
| Glycéryl stéarate | 2 |
| Coco-glucoside (7) | 1 |
| Potasse (KOH) | 6,2 |
| Glycérine | 14 |
| PEG-8 | 7 |
| Methylsilanol/silicate crosspolymer (6) | 3 |
| Conservateurs | 0,9 |
| Eau | Qsp 100 % |

| | |
|---|---|
| (6) NLK 506 de la société Takemoto Oil&fat (7) Plantacare 818 UP de la société Cognis | |

### Exemples 3 et 4 selon l'invention : gel moussant pour le visage

| | Exemple 3 | Exemple 4 |
|---|---|---|
| Coco-bétaine (8) | 6.5 | - |
| Cocoyl glycinate de sodium (9) | 6.5 | - |
| Coco-glucoside (7) | - | 6 |
| Lauryl ether sulfate de sodium (10) | - | 4 |
| PEG-150 pentaérythrityle tétrastéarate (11) | 1 | 1,5 |
| PEG-120 méthylglucose dioléate (12) | 2 | 1 |
| PEG-14M (13) | - | 1 |
| Methylsilanol/silicate crosspolymer (6) | 10 | 5 |
| Eau | Qsp100 % | Qsp100 % |

| | | |
|---|---|---|
| (6) NLK 506 de la société Takemoto Oil&Fat (7) PLANTACARE 818 UP de la société Cognis (8) DEHYTON AB 30 de la société Cognis (9) AMILITE GCS 12 de la société Ajinomoto (10) TEXAPON AOS 225 UP de la société Cognis (11) CROTH IX de la société Croda (12) GLUCAMATE DOE 120 de la société Noveon (13) POLYOX WSR205 de la société Amerchol | | |

### Exemple 5 selon l'invention : shampooing

| | Exemple 6 |
|---|---|
| Lauryléthersulfate de sodium (10) | 15,5 |
| Cocoamphodiacétate (14) | 3,2 |
| Polyquaternium-6 (15) | 0,6 |
| Cétostéarylsulfate de sodium | 0,75 |
| Alcool décylique (C₁₀/C₁₂/C₁₄) oxyéthyléné | 0,5 |
| Methylsilanol/silicate crosspolymer (6) | 5 |
| Acide citrique | qs pH 5 |
| Eau déminéralisée qs | qs100 |

| | |
|---|---|
| (6) NLK 506 de la société Takemoto Oil&Fat (10) TEXAPON AOS 225 UP de la société Cognis (14) MIRANOL C2M CONC de la société Rhodia (15) MERQUAT® 100 de la société CALGON | |

### Exemple 6 selon l'invention et exemples comparatifs 2 et 3

| Composition | Exemple selon l'invention | Exemple comparatif 2 | Exemple comparatif 3 |
|---|---|---|---|
| Lauryl phosphate (1) | 6,5 | 6,5 | 6,5 |
| Potasse (KOH) | 1,7 | 1,7 | 1,7 |
| Disodium EDTA | 0,0495 | 0,0495 | 0,0495 |
| Decyl glucoside (2) | 6,5 | 6, | 6,5 |
| Polyquaternium-7 (3) | 0,5 | 0,5 | 0,5 |
| PEG-60 hydrogenated castor oil (4) | 0,2 | 0,2 | 0,2 |
| PEG-200 glyceryl stearate (5) | 0,5 | 0,5 | 0,5 |
| Chlorure de sodium | 0,1 | 0,1 | 0,1 |
| Sorbitol | 3,5 | 3,5 | 3,5 |
| Conservateurs | 0,4 | 0,4 | 0,4 |
| Glycérine | 3,5 | 3,5 | 3,5 |
| Ethanol | 0,4 | 0,4 | 0,4 |
| Parfum | 0,2 | 0,2 | 0,2 |
| Methylsilanol/silicat e crosspolymer (6) | 1,25 | - | - |
| Silice (7) | - | 1,25 | - |
| Eau | Qsp 100 % | Qsp 100 % | Qsp 100 % |
| Volume de mousse | 9,3 | 8,5 | 7,5 |
| Taille des bulles | 4,5 | 5,5 | 4,8 |
| Douceur | 8,8 | 7,5 | 7,5 |

| | | | |
|---|---|---|---|
| (1) MAP 20® de la société Kao Chemicals (2) MYDOL 10® de la société Kao Chemicals (3) MERQUAT S® de la société Calgon (4) CREMOPHOR RH 60 de la société BASF (5) SIMULSOL 220 TM de la société Seppic (6) NLK 506 de la société Takemoto Oil&fat (7) Aerosil 200 de la société Degussa-Hüls | | | |

Le tableau ci-dessous montre que l'exemple selon l'invention qui contient des sphères creuses organosiliconées, présente une qualité de mousse supérieure à celle d'une composition n'en contenant pas (exemple comparatif 3) et supérieure à celle d'une composition contenant de la silice (exemple comparatif 2), en particulier une mousse plus dense, plus douce et plus fine (taille des bulles plus petite) et plus volumineuse.

## Revendications

1. Composition aqueuse pour application topique contenant (1) au moins un tensioactif moussant, la quantité de tensioactif(s) moussant étant suffisante pour que la composition ait un effet moussant, (2) des particules concaves ou annulaires de matériau siliconé, notamment sous forme de portions de sphères creuses, et (3) au moins 20 % en poids d'eau par rapport au poids total de la composition.

2. Composition selon la revendication précédente, **caractérisée en ce que** les particules concaves de matériau siliconé ont un diamètre moyen inférieur ou égal à 10 µm.

3. Composition selon la revendication précédente, **caractérisée en ce que** les particules concaves de matériau siliconé ont un diamètre moyen allant de 0,1 µm à 8 µm,

4. Composition selon l'une quelconque des revendications précédentes, **caractérisée en ce que** les particules concaves de matériau siliconé sont sous forme de portions de sphères creuses ayant une section transversale en forme de fer à cheval ou d'arceau.

5. Composition selon l'une quelconque des revendications précédentes, **caractérisée en ce que** le matériau siliconé est un polysiloxane réticulé de structure tridimensionnelle comprenant, ou constitué de, des motifs de formule (I) : SiO₂ et de formule (II) : R¹SiO_{1,5} dans lesquelles R¹ désigne un groupe organique ayant un atome de carbone directement relié à l'atome de silicium.

6. Composition selon la revendication précédente, **caractérisée en ce que** R¹ est un groupe alkyle en C₁-C₄ ou un groupe phényle.

7. Composition selon la revendication précédente, **caractérisée en ce que** R¹ est un groupe méthyle.

8. Composition selon la revendication 5, **caractérisée en ce que** R¹ est choisi parmi les groupe époxy, (méth)acryloxy, alkényle, mercaptoalkyle, aminoalkyle, halogénoalkyle, glycéroxy, uréido, cyano.

9. Composition selon l'une quelconque des revendications 5 à 8, **caractérisée en ce que** le matériau siliconé comprend les motifs (I) et (II) selon un rapport molaire motif (I) / motif (II) allant de 30/70 à 50/50, de préférence allant de 35/65 à 45/55.

10. Composition selon l'une quelconque des revendications précédentes, **caractérisée en ce que** les particules concaves en matériau siliconé sont susceptibles d'être obtenues selon un procédé comprenant :
(a) l'introduction dans un milieu aqueux, en présence d'au moins un catalyseur d'hydrolyse, et éventuellement d'au moins un tensioactif, d'un composé (III) de formule SiX₄ et d'un composé (IV) de formule RSiY₃, où X et Y désignent indépendamment l'un de l'autre un groupe alcoxy en C₁-C₄, un groupe alcoxyéthoxy renfermant un groupement alcoxy en C₁-C₄, un groupe acyloxy en C₂-C₄, un groupe N,N-dialkylamino renfermant des groupements alkyle en C₁-C₄, un groupe hydroxyle, un atome d'halogène ou un atome d'hydrogène, et R désigne un groupe organique comportant un atome de carbone directement relié à l'atome de silicium ; et
(b) la mise en contact du mélange résultant de l'étape (a) avec une solution aqueuse renfermant au moins un catalyseur de polymérisation et éventuellement au moins un tensioactif, à une température comprise entre 30 et 85°C, pendant au moins deux heures.

11. Composition selon la revendication précédente, **caractérisée en ce que** dans l'étape (a), le rapport molaire du composé (III) au composé (IV) va de 30/70 à 50/50.

12. Composition selon la revendication 10 ou 11, **caractérisée en ce que** le rapport en poids de l'eau sur le total des composés (III) et (IV) va de 10/90 à 70/30 dans l'étape (a).

13. Composition selon la revendication 10, **caractérisée en ce que** R est un groupe alkyle en C₁-C₄, ou un groupe phényle.

14. Composition selon la revendication précédente, **caractérisée en ce que** R est un groupe méthyle.

15. Composition selon la revendication 10, **caractérisée en ce que** R est choisi parmi les groupes époxy, (méth)acryloxy, alkényle, mercaptoalkyle, aminoalkyle, halogénoalkyle, glycéroxy, uréido, cyano.

16. Composition selon l'une quelconque des revendications précédentes, **caractérisée en ce que** les particules concaves sont formées d'un petit arc interne (11), d'un grand arc externe (21) et de segments (31) qui relient les extrémités des arcs respectifs, la largeur (W1) entre les deux extrémités du petit arc interne (11) allant de 0,01 à 8 µm, de préférence de 0,02 à 6 µm en moyenne, la largeur (W2) entre les eux extrémités du grand arc externe (21) allant de 0,05 à 10 µm, de préférence de 0,06 à 8 µm en moyenne et la hauteur (H) du grand arc externe (21) allant de 0,015 à 8 µm, de préférence de 0,03 à 6 µm en moyenne.

17. Composition selon la revendication 1, **caractérisée en ce que** les particules de forme annulaire présentent un diamètre moyen extérieur de 0,05 à 15 µm et un diamètre moyen intérieur de 001 à 10 µm; la différence entre le diamètre moyen extérieur et le diamètre moyen intérieur étant de 0,04 à 5µm.

18. Composition selon la revendication précédente, présentent un réseau polysiloxane comprenant des unités siloxane de formule (1), (2) (3) (4) (5) et (6)
SiO_{4/2} (1)
Si(OH)_{3/2} (2)
R₁(SiO_{3/2} (3)
R₂SiO_{3/2} (4)
R₃SiO_{3/2} (5)
R₄SiO_{3/2} (6)
dans lesquelles R₁ et R₃ désignent des groupes alkyle, cycloalkyle, aryle, alkylaryle ou aralkyle, et R₂ et R₄ désignent chacun un groupe hydrocarboné choisi parmi les groupes acryloxy, methacryloxy, vinyle ou mercapto ;
le rapport molaire unités siloxane de formule (1) / unités siloxane de formule (2), (3), (4), (5) et (6) étant de 20/80 à 50/50 ;
le rapport molaire unités siloxane de formule (2), (3) et (4) / unités siloxane de formule 5) et (6) étant de 50/50 à 75/25;
le rapport molaire unités siloxane de formule (3) et (5) / unités siloxane de formule (4) et (6) étant de 20/80 à 60/40.

19. Composition selon l'une quelconque des revendications précédentes, **caractérisée en ce que** les particules concaves ou annulaires sont présentes en une teneur allant de 0,1 à 15 % en poids par rapport au poids total de la composition.

20. Composition selon l'une quelconque des revendications précédentes, **caractérisée en ce que** le tensioactif moussant est choisi parmi les tensioactifs non ioniques, les tensioactifs anioniques, les tensioactifs cationiques, les tensioactifs amphotères et zwitterioniques et leurs mélanges.

21. Composition selon l'une quelconque des revendications précédentes, **caractérisée en ce que** le tensioactif moussant est choisi parmi les alkylpolyglucosides, les dérivés de bétaïne, les savons de métal alcalin ou alcalino-terreux ou de bases organiques, les acides alkyl glycol carboxyliques et leurs sels, les alkyl éthers sulfates, les alkylphosphates, les amphodiacétates, les amphoacétates, les alkylglycinates, les acylglutamates, les acylsarcosinates et leurs mélanges.

22. Composition selon l'une quelconque des revendications précédentes, **caractérisée en ce que** la quantité de tensioactif(s) moussant(s) va de 1 à 50 % en poids et de préférence de 2 à 40 % en poids par rapport au poids total de la composition.

23. Composition selon l'une quelconque des revendications précédentes, **caractérisée en ce qu'**elle contient au moins 20 % en poids d'eau par rapport au poids total de la composition.

24. Utilisation cosmétique de la composition selon l'une quelconque des revendications précédentes, pour le nettoyage des matières kératiniques.

25. Procédé cosmétique de nettoyage de la peau, consistant à appliquer sur la peau, une composition selon l'une quelconque des revendications 1 à 23, et à rincer la peau.
